# FASCICULE DE BREVET EUROPEEN

(11) **EP 3 989 979 B1**
(45) Date de publication et mention de la délivrance du brevet: **12.06.2024**
(21) Numéro de dépôt: 20747048.5
(22) Date de dépôt: 26.06.2020
(51) Int. Cl.: A61K 31/69, A61P 35/00, A61P 37/00

(54) **INHIBITEURS DE L'ENTREE CAPACITIVE DU CALCIUM**
KAPAZITIVE CALCIUMEINGANGSINHIBITOREN
CAPACITATIVE CALCIUM ENTRY INHIBITORS

(30) Priorité: 28.06.2019 FR 1907184
(43) Date de publication de la demande: 04.05.2022
(73) Titulaire: Université Paris-Saclay, 91190 Gif-sur-Yvette (FR); INSTITUT NATIONAL DE LA SANTE ET DE LA RECHERCHE MEDICALE - INSERM, 75013 Paris (FR); Sorbonne Université, 75006 Paris (FR); Centre national de la recherche scientifique, 75016 Paris (FR); Université de Bordeaux, 33000 Bordeaux (FR); Bordeaux INP, 33405 Talence (FR)
(72) Inventeur: DELLIS, Olivier, 75013 PARIS (FR); PUCHEAULT, Mathieu, 33360 CAMBLANES-ET-MEYNAC (FR); LE GUILCHER, Camille, 75014 PARIS (FR)
(74) Mandataire: Cabinet Becker et Associés
(86) Numéro de dépôt international: PCT/FR2020/051121
(87) Numéro de publication internationale: WO 2020/260840

(56) Documents cités:
- HOFER ET AL.: BIOORGANIC & MÉDICINAL CHEMISTRY, vol. 21, 2013, pages 3202-3213, XP055560485, cité dans la demande

## Description

La présente invention concerne des inhibiteurs sélectifs de l'entrée capacitive du calcium dépendant d'Orail et leur utilisation thérapeutique.

Le contrôle de l'homéostasie du calcium est un processus complexe essentiel au niveau cellulaire, dans la mesure où l'augmentation de la concentration en ion calcium intracellulaire est impliquée dans la régulation de nombreuses fonctions, dont la contraction, la différenciation et la prolifération. Cette augmentation est due à une libération d'ions calcium à partir de réserves internes, majoritairement à partir du réticulum endoplasmique (RE), et à un influx d'ions calcium depuis le milieu extracellulaire.

Les canaux calciques dits SOCC (pour « Store-Operated Calcium Channels ») représentent la principale voie d'influx du calcium dans les cellules immunitaires dont les leucocytes (ils sont aussi appelés canaux « CRAC » pour Calcium Release Activated Calcium chez les lymphocytes et les mastocytes uniquement). L'influx calcique est contrôlé par deux protéines, STIM1 et Orai1. STIM1 est une protéine située dans la membrane du RE qui agit comme un senseur du calcium présent dans la lumière du RE. Orail est une protéine transmembranaire présente dans la membrane plasmique des leucocytes. Une diminution de la concentration en calcium dans les réserves du RE entraîne une oligomérisation de STIM1, qui interagit dans ces conditions avec les protéines Orail. Cette interaction permet alors l'ouverture des canaux Orail entrainant un influx massif d'ions calcium du milieu extracellulaire. Ce phénomène d'entrée du calcium contrôlée par la concentration calcique dans le RE est appelée SOCE (pour "store-operated calcium entry"). D'autres canaux comme les 2 orthologues Orai2 et Orai3 et certains canaux de la famille TRP forment des SOCE indépendant d'Orail dans d'autres types cellulaires. Ils peuvent aussi servir de protéines associées à Orai1 et modifier les propriétés cinétiques et pharmacologiques des canaux Orai1, mais les protéines Orai1 restent celles qui forment le pore du canal.

Orail a été identifiée suite à la découverte d'une mutation ponctuelle dans sa séquence, résultant en un canal calcique non-fonctionnel dans les cellules leucocytaires, et en un déficit immunitaire combiné sévère. Les canaux formés par Orail jouent donc un rôle crucial dans les fonctions leucocytaires et démontrent que le SOCE des leucocytes est directement dépendant de la présence et de la fonctionnalité d'Orail.

Des composés inhibiteurs des canaux CRAC dépendant d'Orai1, et donc du SOCE dépendant d'Orai1 (ci-après "Orai1-SOCE") pourraient donc être avantageusement utilisés pour contrôler l'activation des cellules immunitaires. Le 2-aminoéthoxy diphénylborate (2-APB) a été décrit comme un modulateur d'Orai1-SOCE, cependant, son activité dépend de la dose utilisée. Entre 1 et 5 µM, le 2-APB potentialise Orai1-SOCE dans les leucocytes, mais l'inhibe à une concentration supérieure à 30 µM (Djillani et al., Biochim Biophys Acta 2014, 1843, 2341-7; Prakriya et al., J Physiol 2001, 536, 3-19). Par ailleurs, il a été rapporté que le 2-APB inhibe le récepteur à l'inositol 1,4,5-triphosphate (ou IP3R; Ma et al., J Biol Chem 2002, 277, 6915-22; Bootman et al., FASEB J 2002, 16, 1145-50) et qu'il inhibe également les pompes à calcium du RE (ou SERCA) (Missiaen et al., Cell Calcium 2001, 29, 111-6; Peppiatt et al., Cell Calcium 2003, 34, 97-108). L'inositol 1,4,5-triphosphate (ou IP3) est un messager secondaire dont la synthèse est induite après stimulation d'une cellule par des ligands extracellulaires comme lors de l'interaction du lymphocyte T avec une cellule présentatrice d'antigène. L'IP3 est alors capable d'induire à son tour une libération d'ions calcium à partir du RE, après fixation à l'IP3R présent sur la face cytoplasmique du RE. Le 2-APB est donc un composé complexe sans spécificité, puisque selon la concentration à laquelle il est utilisé, il module de manière difficilement contrôlable l'activité de plusieurs éléments impactant l'homéostasie du calcium cellulaire.

D'autres inhibiteurs du SOCE ont été proposés, notamment les dérivés de 2-APB en tandem DPB162-AE et DPB163-AE (Goto et al., Cell Calcium 2010, 47, 1-10). Cependant, à faible concentration, le composé DPB163-AE active le SOCE (Goto et al., Cell Calcium 2010, 47, 1-10) alors que le composé DPB162-AE affecte également le contenu des réserves endoplasmiques en calcium dans différents types cellulaires (Bittremieux et al., Cell Calcium 2017, 62, 60-70).

Par ailleurs, Hofer et al. (Bioorganic & Médicinal Chemistry 21 (2013) 3202-3213) ont également développé des dérivés de 2-APB inhibiteurs du transport du calcium dépendant de TRPV6. Cette étude a été réalisée dans des cellules HEK293T, qui sont dépourvues d'Orai1-SOCE. L'article d'Hofer et al. ne fournit donc pas d'enseignement pour la synthèse d'inhibiteurs d'Orai1-SOCE.

L'un des objets de la présente invention est de fournir des composés inhibiteurs d'Orail-SOCE qui soient sélectifs à faible dose, rendant ainsi possible leur utilisation en tant que médicament. C'est dans ce contexte que les inventeurs ont pu démontrer qu'une classe spécifique de composés, définie ci-dessous permet l'inhibition sélective d'Orai1-SOCE à faible dose, sans impacter l'IP3R ou SERCA.

Ainsi, l'invention a pour objet un composé de formule (I) ou un sel pharmaceutiquement acceptable de celui-ci, pour son utilisation dans la modulation de la SOCE dépendant d'Orail : 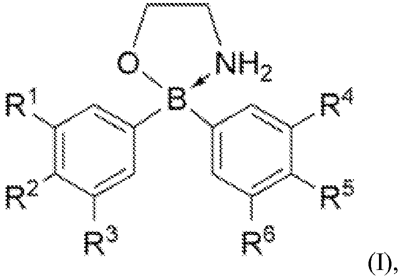
dans laquelle, R1, R2, R3, R4, R5 et R6 sont des groupes identiques ou différents, choisis parmi un atome d'hydrogène, un atome d'halogène, un groupement alkyle en C1-C6, un groupement alcényle en C1-C6, un groupement alkyloxy en C1-C6, un groupement alkylthio en C1-C6, un groupement aryle en C6-C14 et un groupement hétéroaryle comprenant 5 à 14 chaînons;
sous réserve que lorsque deux des groupements R1, R2 et R3 représentent un hydrogène, alors le groupement parmi R1, R2 et R3 qui n'est pas un hydrogène est choisi parmi un groupement alkyle en C3-C6, un groupement alcényle en C3-C6, un groupement alkyloxy en C3-C6, un groupement alkylthio en C3-C6, un groupement aryle en C6-C14 et un groupement hétéroaryle comprenant 5 à 14 chaînons;
sous réserve que lorsque deux des groupements R4, R5 et R6 représentent un hydrogène, alors le groupement parmi R4, R5 et R6 qui n'est pas un hydrogène est choisi parmi un groupement alkyle en C3-C6, un groupement alcényle en C3-C6, un groupement alkyloxy en C3-C6, un groupement alkylthio en C3-C6, un groupement aryle en C6-C14 et un groupement hétéroaryle comprenant 5 à 14 chaînons;
les groupements R1 et R2 pouvant former, avec les atomes de carbone auxquels ils sont liés, un cycle comprenant 5 à 7 chaînons;
les groupements R4 et R5 pouvant former, avec les atomes de carbone auxquels ils sont liés, un cycle comprenant 5 à 7 chaînons; et
lesdits groupements aryles ou hétéroaryles étant susceptibles d'être substitués par un ou plusieurs substituants choisis parmi les atomes d'halogène, les groupements alkyles en C1-C6, les groupements alcényles en C2-C6, les groupements alkyloxy en C1-C6, les groupements alkylthio en C1-C6, les groupements cétones en C2-C6, les groupements esters en C1-C6, les groupements aldéhydes en C1-C6, -NH2, -CN, -CF₃, les groupements amine en C1-C6 et les groupements amine en C1-C6 mono- ou di-substitués par un groupement alkyle en C1-C6.

L'invention concerne également un composé de formule (I), pour son utilisation en tant que médicament.

Par "halogène", on entend un atome de fluor, chlore, brome ou iode.

Par "alkyle", on entend un groupe aliphatique saturé, linéaire ou ramifié. L'expression "alkyle en C1-C6" peut plus particulièrement désigner un groupe méthyle, éthyle, propyle, isopropyle, n-butyle, iso-butyle, sec-butyle, tert-butyle, pentyle, ou hexyle.

Par "alcène", on entend un groupe aliphatique linéaire ou ramifié, comprenant au moins une double liaison carbone-carbone.

Par "alkyloxy", on entend un groupe -O-alkyle où le groupe alkyle est tel que défini ci-dessus. L'expression "alkyloxy en C1-C6" peut plus particulièrement désigner un groupe méthoxy, éthoxy, propyloxy, isopropyloxy, n-butyloxy, iso-butyloxy, tert-butyloxy, sec-butyloxy, pentyloxy, ou hexyloxy.

Par "alkylthio", on entend un groupe -S-(alkyle), où le groupe alkyle est tel que défini ci-dessus. L'expression "alkylthio en C1-C6" peut plus particulièrement désigner un groupe méthylthio, éthylthio, propylthio, isopropylthio, n-butylthio, iso-butylthio, tert-butylthio, sec-butylthio, pentylthio, ou hexylthio.

Par "aryle", on entend un groupe aromatique hydrocarboné monocyclique ou polycyclique. Plus particulièrement, un aryle peut désigner un phényle, un biphényle, ou un naphtyle, et de préférence un phényle.

Par "hétéroaryle", on entend un groupe aromatique monocyclique ou polycyclique, comprenant au moins un hétéroatome tel que l'azote, l'oxygène ou le soufre. Plus particulièrement, un hétéroaryle peut désigner un pyridinyle, thiazolyle, thiophényle, furanyle, pyrrolyle, imidazolyle, triazolyle, tétrazolyle, benzofuranyle, thianaphthalényle, indolyle, indolinyle, quinolinyle, isoquinolinyle, benzimidazolyle, triazinyle, thianthrényle, isobenzofuranyle, phénoxanthinyle, isothiazolyle, isoxazolyle, pyrazinyle, pyridazinyle, indolizinyle, isoindolyle, indazolyle, purinyle, phtalazinyle, naphthyridinyle, quinoxalinyle, quinazolinyle, cinnolinyle, ptéridinyle, carbazolyle, β-carbolinyle, phénanthridinyle, acridinyle, pyrimidinyle, phénanthrolinyle, phénazinyle, phénothiazinyle, furazanyle, phenoxazinyle, benzotriazolyle, benzoxazolyle, benzisoxazolyle, oxindolyle, benzothiényle, benzothiazolyle, s-triazinyle, oxazolyle, ou thiofuranyle.

Selon un mode particulier de réalisation, un groupement parmi R1, R2 et R3 représente un atome d'hydrogène. Selon une variante de ce mode de réalisation, un groupement parmi R1, R2 et R3 représente un atome d'hydrogène et les deux autres groupements parmi R1, R2 et R3 représentent indépendamment un atome d'halogène, un groupement alkyle en C1-C6, un groupement alcényle en C1-C6, un groupement alkyloxy en C1-C6, un groupement alkylthio en C1-C6, un groupement aryle en C6-C14 ou un groupement hétéroaryle comprenant 5 à 14 chaînons. Selon un mode particulier de réalisation, R3 est un atome d'hydrogène et R1 et R2, identiques ou différents, représentent un atome d'halogène, un groupement alkyle en C1-C6, un groupement alcényle en C1-C6, ou un groupement alkyloxy en C1-C6 ou un groupement alkylthio en C1-C6. Selon un autre mode de réalisation, R3 est un atome d'hydrogène et R1 et R2, identiques ou différents, représentent un atome d'halogène, un groupement alkyle en C1-C6, un groupement alcényle en C1-C6 ou un groupement alkyloxy en C1-C6. De manière encore plus particulière, R3 peut être un atome d'hydrogène et R1 et R2, identiques ou différents, peuvent représenter un atome d'halogène, un groupement alkyle en C1-C6 ou un groupement alkyloxy en C1-C6. Selon un autre mode particulier de réalisation, R3 est un atome d'hydrogène et R1 et R2, identiques ou différents, représentent un atome d'halogène ou un groupement alkyle en C1-C6. Selon un mode préféré de réalisation R3 représente un atome d'hydrogène, R1 représente un atome d'halogène ou un groupement alkyle en C1-C6, et R2 représente un atome d'halogène ou un groupement alkyle en C1-C6. Selon une variante de réalisation, R3 représente un atome d'hydrogène, R1 représente un atome d'halogène, et R2 représente un groupement alkyle en C1-C6. Selon une autre variante, R3 représente un atome d'hydrogène, R1 représente un groupement alkyle en C1-C6, et R2 représente un atome d'halogène. Selon encore une autre variante, R3 représente un atome d'hydrogène, R1 représente un groupement alkyle en C1-C6, notamment méthyle, et R2 représente un groupement alkyle en C1-C6, notamment méthyle. Plus particulièrement, R3 représente un atome d'hydrogène, R1 représente un atome d'halogène, notamment de chlore, et R2 représente un groupement méthyle. Dans un autre mode particulier de réalisation, R3 représente un atome d'hydrogène, R1 représente un groupement méthyle et R2 représente un atome d'halogène, notamment de chlore.

Selon un mode particulier de réalisation, R2 est un atome d'hydrogène et R1 et R3, identiques ou différents, représentent un atome d'halogène, un groupement alkyle en C1-C6, un groupement alcényle en C1-C6, ou un groupement alkyloxy en C1-C6 ou un groupement alkylthio en C1-C6. Selon un autre mode de réalisation, R2 est un atome d'hydrogène et R1 et R3, identiques ou différents, représentent un atome d'halogène, un groupement alkyle en C1-C6, un groupement alcényle en C1-C6 ou un groupement alkyloxy en C1-C6. De manière encore plus particulière, R2 peut être un atome d'hydrogène et R1 et R3, identiques ou différents, peuvent représenter un atome d'halogène, un groupement alkyle en C1-C6 ou un groupement alkyloxy en C1-C6. Selon un autre mode particulier de réalisation, R2 est un atome d'hydrogène et R1 et R3, identiques ou différents, représentent un atome d'halogène ou un groupement alkyle en C1-C6. Selon un mode préféré de réalisation R2 représente un atome d'hydrogène, R1 représente un atome d'halogène ou un groupement alkyle en C1-C6, et R3 représente un atome d'halogène ou un groupement alkyle en C1-C6. Selon une variante de réalisation, R2 représente un atome d'hydrogène, R1 représente un atome d'halogène, et R3 représente un groupement alkyle en C1-C6. Selon une autre variante, R2 représente un atome d'hydrogène, R1 représente un groupement alkyle en C1-C6, et R3 représente un atome d'halogène. Selon encore une autre variante, R2 représente un atome d'hydrogène, R1 représente un groupement alkyle en C1-C6, notamment méthyle, et R3 représente un groupement alkyle en C1-C6, notamment méthyle. Plus particulièrement, R2 représente un atome d'hydrogène, R1 représente un atome d'halogène, notamment de chlore, et R3 représente un groupement méthyle. Dans un autre mode particulier de réalisation, R2 représente un atome d'hydrogène, R1 représente un groupement méthyle et R3 représente un atome d'halogène, notamment de chlore.

Selon un autre mode particulier de réalisation, un groupement parmi R4, R5 et R6 représente un atome d'hydrogène. Selon une variante de ce mode de réalisation, un groupement parmi R4, R5 et R6 représente un atome d'hydrogène et les deux autres groupements parmi R4, R5 et R6 représentent indépendamment un atome d'halogène, un groupement alkyle en C1-C6, un groupement alcényle en C1-C6, un groupement alkyloxy en C1-C6, un groupement alkylthio en C1-C6, un groupement aryle en C6-C14 ou un groupement hétéroaryle comprenant 5 à 14 chaînons. Selon un mode particulier de réalisation, R6 est un atome d'hydrogène et R4 et R5, identiques ou différents, représentent un atome d'halogène, un groupement alkyle en C1-C6, un groupement alcényle en C1-C6, ou un groupement alkyloxy en C1-C6 ou un groupement alkylthio en C1-C6. Selon un autre mode de réalisation, R6 est un atome d'hydrogène et R4 et R5, identiques ou différents, représentent un atome d'halogène, un groupement alkyle en C1-C6, un groupement alcényle en C1-C6 ou un groupement alkyloxy en C1-C6. De manière encore plus particulière, R6 peut être un atome d'hydrogène et R4 et R5, identiques ou différents, peuvent représenter un atome d'halogène, un groupement alkyle en C1-C6 ou un groupement alkyloxy en C1-C6. Selon un autre mode particulier de réalisation, R6 est un atome d'hydrogène et R4 et R5, identiques ou différents, représentent un atome d'halogène ou un groupement alkyle en C1-C6. Selon un mode préféré de réalisation R6 représente un atome d'hydrogène, R4 représente un atome d'halogène ou un groupement alkyle en C1-C6, et R5 représente un atome d'halogène ou un groupement alkyle en C1-C6. Selon une variante de réalisation, R6 représente un atome d'hydrogène, R4 représente un atome d'halogène, et R5 représente un groupement alkyle en C1-C6. Selon une autre variante, R6 représente un atome d'hydrogène, R4 représente un groupement alkyle en C1-C6, et R5 représente un atome d'halogène. Selon encore une autre variante, R6 représente un atome d'hydrogène, R4 représente un groupement alkyle en C1-C6, notamment méthyle, et R5 représente un groupement alkyle en C1-C6, notamment méthyle. Plus particulièrement, R6 représente un atome d'hydrogène, R4 représente un atome d'halogène, notamment de chlore, et R5 représente un groupement méthyle. Dans un autre mode particulier de réalisation, R6 représente un atome d'hydrogène, R4 représente un groupement méthyle et R5 représente un atome d'halogène, notamment de chlore.

Selon un mode particulier de réalisation, R5 est un atome d'hydrogène et R4 et R6, identiques ou différents, représentent un atome d'halogène, un groupement alkyle en C1-C6, un groupement alcényle en C1-C6, ou un groupement alkyloxy en C1-C6 ou un groupement alkylthio en C1-C6. Selon un autre mode de réalisation, R5 est un atome d'hydrogène et R4 et R6, identiques ou différents, représentent un atome d'halogène, un groupement alkyle en C1-C6, un groupement alcényle en C1-C6 ou un groupement alkyloxy en C1-C6. De manière encore plus particulière, R5 peut être un atome d'hydrogène et R4 et R6, identiques ou différents, peuvent représenter un atome d'halogène, un groupement alkyle en C1-C6 ou un groupement alkyloxy en C1-C6. Selon un autre mode particulier de réalisation, R5 est un atome d'hydrogène et R4 et R6, identiques ou différents, représentent un atome d'halogène ou un groupement alkyle en C1-C6. Selon un mode préféré de réalisation R5 représente un atome d'hydrogène, R4 représente un atome d'halogène ou un groupement alkyle en C1-C6, et R6 représente un atome d'halogène ou un groupement alkyle en C1-C6. Selon une variante de réalisation, R5 représente un atome d'hydrogène, R4 représente un atome d'halogène, et R6 représente un groupement alkyle en C1-C6. Selon une autre variante, R5 représente un atome d'hydrogène, R4 représente un groupement alkyle en C1-C6, et R6 représente un atome d'halogène. Selon encore une autre variante, R5 représente un atome d'hydrogène, R4 représente un groupement alkyle en C1-C6, notamment méthyle, et R6 représente un groupement alkyle en C1-C6, notamment méthyle. Plus particulièrement, R5 représente un atome d'hydrogène, R4 représente un atome d'halogène, notamment de chlore, et R6 représente un groupement méthyle. Dans un autre mode particulier de réalisation, R5 représente un atome d'hydrogène, R4 représente un groupement méthyle et R6 représente un atome d'halogène, notamment de chlore.

Selon un autre mode de réalisation, deux groupements parmi R1, R2 et R3 représentent un atome d'hydrogène, et l'autre groupement parmi R1, R2 et R3 représente un groupement un groupement alkyle en C3-C6, un groupement alcényle en C3-C6, un groupement alkyloxy en C3-C6, un groupement alkylthio en C3-C6, un groupement aryle en C6-C14 ou un groupement hétéroaryle comprenant 5 à 14 chaînons. Plus particulièrement, dans une autre variante, le groupement parmi R1, R2 et R3 qui n'est pas un atome d'hydrogène est choisi parmi un groupement alkyle en C3-C6, un groupement aryle en C6-C14 et un groupement hétéroaryle comprenant 5 à 14 chaînons. Encore plus particulièrement, le groupement parmi R1, R2 et R3 qui n'est pas un atome d'hydrogène est choisi parmi un groupement alkyle en C3-C6 et un groupement aryle en C6-C14. Dans Selon un mode particulier de réalisation, le groupement alkyle en C3-C6 est un groupement alkyle en C3-C5. Préférentiellement, les deux groupements qui représentent des hydrogènes sont R1 et R3. Dans ce mode préférentiel, R2 peut plus particulièrement être choisi parmi les groupements n-butyle, isobutyle ou tert-butyle, plus particulièrement n-butyle, et un groupement aryle, notamment phényle.

Selon un autre mode de réalisation, deux groupements parmi R4, R5 et R6 représentent un atome d'hydrogène, et l'autre groupement parmi R4, R5 et R6 représente un groupement un groupement alkyle en C3-C6, un groupement alcényle en C3-C6, un groupement alkyloxy en C3-C6, un groupement alkylthio en C3-C6, un groupement aryle en C6-C14 ou un groupement hétéroaryle comprenant 5 à 14 chaînons. Plus particulièrement, dans une autre variante, le groupement parmi R4, R5 et R6 qui n'est pas un atome d'hydrogène est choisi parmi un groupement alkyle en C3-C6, un groupement aryle en C6-C14 et un groupement hétéroaryle comprenant 5 à 14 chaînons. Encore plus particulièrement, le groupement parmi R4, R5 et R6 qui n'est pas un atome d'hydrogène est choisi parmi un groupement alkyle en C3-C6 et un groupement aryle en C6-C14. Dans Selon un mode particulier de réalisation, le groupement alkyle en C3-C6 est un groupement alkyle en C3-C5. Préférentiellement, les deux groupements qui représentent des hydrogènes sont R4 et R6. Dans ce mode préférentiel, R5 peut plus particulièrement être choisi parmi les groupements n-butyle, isobutyle, sec-butyle ou tert-butyle, plus particulièrement n-butyle, et un groupement aryle, notamment phényle.

Selon un autre mode particulier de réalisation, R1, R3, R4 et R6 représentent un atome d'hydrogène. Selon une variante de réalisation, R1, R3, R4 et R6 représentent un atome d'hydrogène et R2 et R5, identiques ou différents, identiques de préférence, représentent un groupement alkyle en C3-C6, notamment un groupement n-butyle, isobutyle, sec-butyle ou tert-butyle, plus particulièrement n-butyle, ou un groupement aryle en C6-C14, plus particulièrement un groupement aryle en C6-C8. Préférentiellement, R2 et R5 sont identiques et représentent un groupement phényle non substitué, ou un groupement phényle substitué par un ou plusieurs substituants choisis parmi les atomes d'halogène, les groupements alkyles en C1-C6, les groupements alcényles en C2-C6, les groupements alkyloxy en C1-C6, les groupements alkylthio en C1-C6, les groupements cétones en C2-C6, les groupements esters en C1-C6, les groupements aldéhydes en C1-C6, -NH2, -CN, -CF₃, les groupements amine en C1-C6 et les groupements amine en C1-C6 mono- ou di-substitués par un groupement alkyle en C1-C6, le groupement phényle pouvant plus particulièrement être substitué par un ou plusieurs substituants choisis parmi les atomes d'halogène, les groupements alkyles en C1-C6, les groupements alcènes en C2-C6 et les groupements alkyloxy. Selon un mode préféré de réalisation, R2 et R5 sont identiques et représentent un groupement phényle non-substitué.

Selon un autre mode de réalisation, les groupements R1 et R2 peuvent former, avec les atomes de carbone auxquels ils sont liés, un cycle comprenant 5 à 7 chaînons. Ainsi, on peut notamment citer un cycle formant, avec le groupement phényle auquel il est lié, un groupement naphtyle ou benzothiényle (modes de réalisation illustrés par les composés P6 et P12). De même, les groupements R4 et R5 peuvent former, avec les atomes de carbone auxquels ils sont liés, un cycle comprenant 5 à 7 chaînons, ce cycle pouvant notamment former, avec le groupement phényle auquel il est lié, un groupement naphtyle ou benzothiényle.

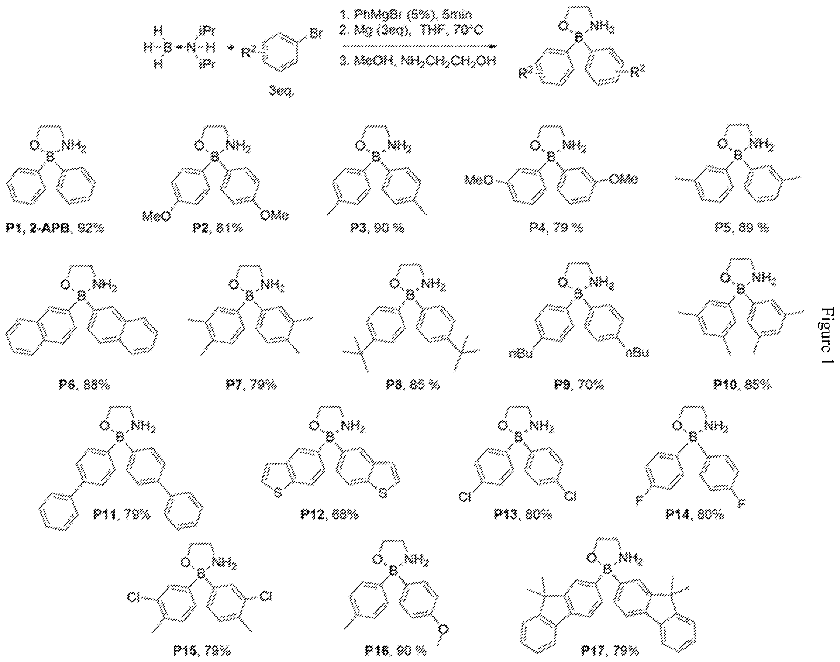

Selon un autre mode avantageux de réalisation, le composé de formule (I) est choisi parmi les composés de la figure 1. Selon un mode particulièrement préféré, le composé de formule (I) est choisi parmi les composés P11, P9, P6, P15, P8, P12, P10 et P7 de la figure 1. Dans un mode préféré de réalisation, le composé de formule (I) est choisi par la 2-(dibiphényl-4-ylboryloxy)éthamine (composé P11) et la 2-(bis(4-(tert-butyl)phényl)boryl)oxy)éthanamine (composé P9). Encore plus avantageusement, le composé de formule (I) est le composé P11.

La présente invention vise également les sels pharmaceutiquement acceptables des composés de formule (I), et leur utilisation. D'une manière générale, ce terme désigne les sels peu ou non toxiques obtenus à partir de bases ou d'acides, organiques ou inorganiques. Ces sels peuvent être obtenus lors de l'étape de purification finale du composé selon l'invention ou par incorporation du sel sur le composé déjà purifié.

Les composés de formule (I) peuvent être préparés selon des méthodes connues de l'homme du métier, qui pourra notamment se référer aux enseignements des documents EP1444981 et FR3021050. La synthèse du composé P11 est notamment décrite dans l'exemple 16 du document FR3021050. D'autres procédés utiles pour la synthèse des composés de formule (I) ont été décrits par Richard et al., Synthesis 2017, 49, p. 736-744.

Un composé de formule (I) peut être formulé sous la forme d'une composition pharmaceutique. Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, les liposomes, etc. Les compositions peuvent être formulées sous forme de suspensions injectables, gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons. Les composés ou compositions selon l'invention peuvent être administrés de différentes manières et sous différentes formes. Ainsi, ils peuvent être par exemple administrés de manière systémique, par voie orale, parentérale, par inhalation ou par injection, comme par exemple par voie intraveineuse, intra-musculaire, sous-cutanée, trans-dermique, intra-artérielle, etc. Pour les injections, les composés sont généralement conditionnés sous forme de suspensions liquides, qui peuvent être injectées au moyen de seringues ou de perfusions, par exemple.

Il est ici démontré que les composés de formule (I) sont capables d'inhiber la SOCE avec un Ki submicromolaire, et que cette activité inhibitrice est sélective d'Orail-SOCE à faible dose, par comparaison à son effet contre l'IP3R et les pompes SERCA. Les composés de formule (I), plus particulièrement les composés P9 et P11, sont donc particulièrement utiles pour inhiber l'Orai1-SOCE. L'invention a donc pour objet un composé de formule (I), pour son utilisation dans l'inhibition d'Orail-SOCE. L'invention a donc également pour objet un composé de formule (I) pour son utilisation dans la modulation des canaux calciques dans une cellule immunitaire.

Plus généralement, l'invention a pour objet un composé de formule (I), pour son utilisation en tant que médicament, notamment en tant qu'immunosuppresseur. Dans ces conditions, les composés de formule (I) peuvent ainsi notamment être utilisés afin de prévenir un rejet de greffe.

L'invention a également pour objet un composé de formule (I) pour le traitement d'une hypertension artérielle pulmonaire.

L'invention vise plus particulièrement un composé de formule (I) pour son utilisation en tant qu'inhibiteur des cellules immunitaires, notamment les leucocytes tels que les lymphocytes T et lymphocytes B, ainsi que les monocytes.

L'invention concerne également un composé de formule (I) pour son utilisation dans l'inhibition des fonctions des cellules immunitaires. A titre illustratif, on peut citer leur utilisation pour inhiber la production de cytokines, notamment de cytokines qui régulent l'activation des cellules immunitaires, plus particulièrement pour inhiber la production de l'IL-2 par les lymphocytes T. On peut également citer leur utilisation pour inhiber la production de radicaux oxygénés libres par les neutrophiles ou pour inhiber la phagocytose par les macrophages (fonctions impliquées dans les inflammations chroniques)

Les composés de formule (I) peuvent être utilisés pour le traitement d'un trouble pour lequel l'utilisation d'un inhibiteur de Orail-SOCE est indiquée. Le terme "traitement" désigne le traitement curatif, symptomatique ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression de la maladie, ou prévenir une progression plus en avant de la maladie, améliorant ainsi la condition des sujets. Les composés de la présente invention peuvent enfin être administrés aux sujets non malades, mais qui pourraient développer normalement la maladie ou qui ont un risque important de développer la maladie.

A ce titre, l'invention vise notamment un composé de formule (I), pour son utilisation dans une méthode de traitement d'un trouble inflammatoire, notamment un trouble inflammatoire chronique ou aigu, plus particulièrement chronique.

L'invention concerne également un composé de formule (I) pour son utilisation dans le traitement d'un trouble allergique.

L'invention vise par ailleurs un composé de formule (I) pour son utilisation dans une méthode de traitement d'un trouble immunitaire. Parmi les troubles immunitaires susceptibles d'être traités au moyen du composé de formule (I), on peut notamment citer les maladies auto-immunes et les maladies inflammatoires, plus particulièrement les maladies inflammatoires chroniques. A titre illustratif, le composé de formule (I) peut être utilisé dans le traitement de maladies inflammatoires chroniques telles que l'arthrite rhumatoïde, la pancréatite, les inflammations de l'intestin comme la maladie de Crohn ou la rectite, le psoriasis ou encore le diabète de type 2. Par ailleurs les maladies auto-immunes susceptibles d'être traitées au moyen du composé de formule (I) correspondent à toutes les maladies impliquant une action excessive du système immunitaire contre une cellule, un tissu, ou un organe du sujet à traiter. On peut citer à titre illustratif le psoriasis, le lupus, les vascularites, la polyarthrite rhumatoïde, la sclérose en plaques, le vitiligo, la sclérodermie, le diabète de type 1 ou les hépatites auto-immunes.

L'invention concerne également un composé de formule (I), pour son utilisation dans le traitement d'un cancer. L'invention vise plus particulièrement le traitement d'un cancer impliquant Orail. Plus particulièrement, l'invention vise un composé de formule (I) pour son utilisation dans le traitement d'un cancer du sang, notamment d'une leucémie ou d'un lymphome, ou d'un cancer du sein. Selon un autre mode particulier de réalisation, l'invention vise un composé de formule (I) pour son utilisation dans le traitement d'un cancer hormono-indépendant, notamment un cancer du sein hormono-indépendant. Dans le contexte de la présente invention, un cancer est qualifié de "cancer hormono-indépendant" si des hormones féminines (notamment oestrogènes ou progestérone), ne jouent pas un rôle dans la prolifération des cellules cancéreuses. L'homme du métier est en mesure de déterminer si un cancer est hormono-indépendant, notamment par examen anatomopathologique.

L'invention concerne par ailleurs un composé de formule (I), pour son utilisation dans le traitement de maladies dites « à gain de fonction » comme le syndrome de Stormorken ou la myopathie à agrégats tubulaires due à des mutations du gène Orail aboutissant à une suractivité des canaux Orail et un SOCE constitutif.

L'invention concerne également une méthode de traitement d'un trouble pour lequel l'utilisation d'un inhibiteur de Orail-SOCE est indiquée, plus particulièrement l'un des troubles mentionnés ci-dessus, comprenant l'administration d'une quantité efficace d'un composé de formule (I). Au sens de l'invention le terme "une quantité efficace" se réfère à une quantité du composé suffisante pour produire le résultat biologique désiré. Au sens de la présente invention le terme "sujet" signifie un mammifère et plus particulièrement un humain. La quantité de composé de formule (I) à administrer peut varier dans une large mesure, et dépendra notamment du trouble à traiter et de son stade, de l'âge, du sexe ou du poids du sujet à traiter, de la voie d'administration et/ou de la durée du traitement. Dans le cas d'un sujet humain, la dose administrée oralement peut notamment être comprise entre 1 mg et 4000 mg, notamment entre 1 mg et 1000 mg, une ou plusieurs fois par jour. Une administration par voie parentérale, notamment par voie intravasculaire, plus particulièrement par voie intraveineuse ou intra-artérielle, préférentiellement par voie intraveineuse, pourra notamment se faire à une dose comprise entre 0,1 et 100 mg, une ou plusieurs fois par jour, ou en administration continue pendant 1 à 24 heures, par voie intraveineuse.

Les exemples suivants illustre l'invention, sans toutefois la limiter.

### LEGENDE DES FIGURES

La figure 1 représente la structure du composé 2-APB (composé P1) et des analogues de 2-APB divulgués dans la présente demande.
La figure 2 montre que le 2-APB a un effet dual sur le SOCE des cellules T Jurkat, alors que les autres analogues de 2-APB n'ont qu'un effet inhibiteur
La figure 3 est un graphique montrant que le composé P11 inhibe l'influx de Mn²⁺ via les SOCC et le SOCE d'autres types cellulaires.
La figure 4 est un graphique montrant que les composés P9 et P11 inhibent préférentiellement le SOCE par rapport au chargement via SERCA et par rapport à la libération de calcium à partir du RE par l'IP3R ("IICR").
La figure 5 est un graphique montrant que le composé P11 augmente la mort cellulaire et diminue la synthèse d'interleukine-2 par les cellules Jurkat stimulées avec la PHA.
La figure 6 est un graphique montrant que le composé P11 augmente l'activité caspase-3 des cellules Jurkat stimulée avec la PHA.
La figure 7 est un graphique montrant que le composé P11 augmente la fragmentation de l'ADN dans les cellules Jurkat stimulées avec la PHA.

### EXEMPLES

### 1. Matériels et méthodes

Pour caractériser l'effet des molécules sur le SOCE, la lignée de lymphocytes T Jurkat a principalement été utilisée. Pour visualiser les variations de calcium cytosolique, la sonde ratiométrique Indo-1 a été utilisée. Pour cela, les cellules en culture ont été centrifugées puis resuspendues et incubées dans un milieu PBS (Phosphate Buffer Saline) additionné de 1 mg/ml d'albumine et 4 µM d'Indo-1-AM durant 45 min à température ambiante et dans l'obscurité. Puis les cellules ont été à nouveau centrifugées et resuspendues dans le milieu d'enregistrement HBS (Hepes-Buffer Saline) de composition (en mM) : 135 mM NaCl, 5.9 mM KCl, 1.2 mM MgCl₂, 11.6 mM Hepes, 11.5 mM glucose, pH ajusté à 7.3 avec NaOH. 0.5 à 1 million de cellules sont alors placées dans la cuvette d'un spectrofluorimètre Varian Cary Eclipse et la sonde Indo-1 est excitée à 360 nm, avec émission de fluorescence à 405 et 480 nm. Le ratio de fluorescence à 405 et 480 nm est directement proportionnel à la [Ca²⁺]cyt.

Les lignées d'origine monocytaire U937, lymphocytaire B (DG75) et de cancer du sein (MDA-MB231) ont aussi été utilisées avec le même protocole.

Pour tester la sélectivité de certaines molécules sur le SOCE par rapport aux IP3R et SERCA, la lignée L15 exprimant de manière stable l'IP3R de type 1 a été utilisée. Brièvement les cellules ont été perméabilisées par de la saponine et incubées en présence de ⁴⁵Ca²⁺ dans un milieu contenant de l'ATP permettant l'activité des SERCA et l'entrée des ions Ca²⁺ dans le RE. L'ajout de TG permet d'induire un relargage du RE et donc de mesurer la quantité d'ions Ca²⁺ sorti du RE, qui est elle-même directement liée à l'activité des SERCA. L'ajout d'IP3 permet la mesure directe de la quantité d'ions Ca2+ sortant du RE par ouverture des IP3R.

Pour mesurer la synthèse d'interleukine-2 (IL-2) en 24 h après stimulation par 10 µg/ml de phytohemagglutinine (PHA) en présence ou non de certaines molécules, les cellules Jurkat sont centrifugées et le surnageant contenant l'IL-2 est collecté. La mesure de la concentration en Il-2 se fait par Elisa (Kit R&D system).

La mesure de l'activité caspase-3 se fait par un kit fluorescent utilisant le substrat Ac-DEVD-AFC. Les cellules Jurkat sont incubées 24 h avec la PHA en présence ou non de certaines molécules. Puis les cellules sont lysées avec du milieu RIPA, les quantités de protéines sont alors mesurées par un kit commercial (Biorad). Les lysats cellulaires sont alors dilués dans le milieu réactionnel suivant : 200 mM Hepes pH 7.4, 1 mM EDTA, 20% sucrose et 20 mM dithiothreitol, puis incubés avec 20 µM du substrat fluorogénique pendant 45 min à 7 h à 37 °C. L'apparition du produit AFC fluorescent est mesuré par spectrofluorimétrie (excitation à 405 nm, émission à 505 nm)
Pour observer la fragmentation de l'ADN nucléaire et donc l'apoptose cellulaire, le kit TUNEL Roche a été utilisé.

### 2. Résultats

### 2.1. Effet des analogues sur la concentration en calcium cytosolique

De nouveaux analogues de 2-APB ont été synthétisés afin d'identifier des inhibiteurs forts de SOCE. Les molécules testées et leur voie de synthèse sont présentées dans la figure 1.

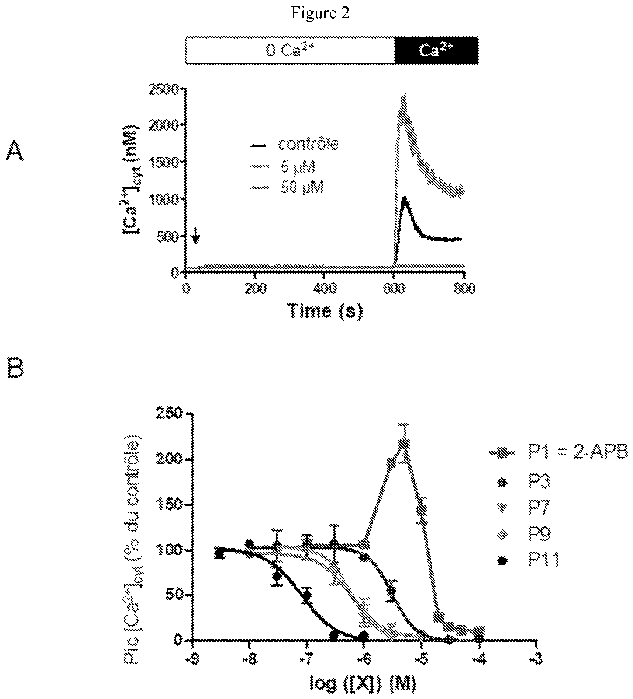

Le phénomène de SOCE a été évalué de manière classique. Des cellules Jurkat ont été placées dans un milieu HBS sans calcium et stimulées avec 1 µM de thapsigargine (TG, flèche dans la figure 2A) pendant 600 s afin de permettre la libération du calcium à partir du RE et l'ouverture des canaux SOCC (= CRAC). L'ajout de 1 mM de CaCl₂ dans le milieu permet une entrée massive d'ions calcium au travers des SOCC et l'augmentation de la concentration en calcium cytosolique ([Ca²⁺]_{cyt}).

La synthèse des nouveaux composés a été validée par l'évaluation de l'effet de 2-APB synthétisé dans les mêmes conditions. La figure 2A montre que le 2-APB ainsi synthétisé possède bien ses propriétés classiques: une potentialisation importante de l'entrée du calcium à 5 µM et une inhibition quasi totale à 50 µM.

Les courbes dose-réponse ont été établies après ajout de 2-APB ou de l'un de ses analogues 30 secondes avant la réintroduction de calcium. Les résultats montrent que les analogues du 2-APB ne présentent pas la propriété d'augmenter la concentration en calcium cytosolique. L'augmentation de la taille des molécules au niveau des groupements phényles a donc un effet négatif sur l'activité de potentialisation. Les analogues testés sont donc des inhibiteurs de SOCE sans l'effet de potentialisation de l'augmentation du calcium cytosolique normalement observé à faible dose avec le 2-APB (cf. tableau 1).

**Tableau 1**

| **Composé** | **Ki (nM)** |
|---|---|
| P11 | 75 ± 21 |
| P6 | 275 ± 17 |
| P15 | 294 ± 74 |
| P8 | 350 ± 40 |
| P12 | 374 ± 96 |
| Dibenzothienyl-APB (Djillani 2014) | 405 ± 23 |
| P10 | 484 ± 116 |
| P9 | 641 ± 103 |
| P7 | 751 ± 97 |

| **Composé** | **Ki (µM)** |
|---|---|
| P13 | 1,84 ± 0,1 |
| P5 | 2,1 ± 0,6 |
| P16 | 3,1 ± 0,6 |
| P3 | 3,5 ± 0,6 |
| P2 | 3,5 ± 0,65 |
| P14 | 5,4 ± 0,3 |
| P4 | 75 ± 21 |
| P17 | >> 1 µM |

Les composés P3 et P5 comprennent un groupe méthyle sur chacun des groupement phényles de la molécule (en para et méta, respectivement). P7 et P10 correspondent aux composés P3 et P5 auxquels un second groupe méthyle a été ajouté sur chacun des groupements phényles (un groupe en meta et un groupe en para de chacun des phényles). Ces modifications résultent en une augmentation significative de l'efficacité d'inhibition des molécules. Le composé P10 est toutefois le plus actif des deux avec un Ki d'environ 500 nM et une inhibition totale obtenue à 3 µM.

D'autres analogues ont été générés avec un groupe méthoxy sur chacun des groupements phényles du 2-APB (composés P2 et P4). On observe que le placement du groupe méthoxy en position para permet l'obtention d'un composé (composé P2) ayant une activité équivalente à celle du composé comprenant un groupe méthyle à cette position (composé P3). Par contre, le placement de ce groupe méthoxy en méta (composé P4) a un impact négatif drastique sur la constante d'inhibition, qui monte à 75 ± 21 µM, avec une inhibition maximale de 80 % seulement. Le composé P16 combine un groupe méthyle en para de l'un des phényles, et un groupe méthoxy sur l'autre phényle. Ce composé à une activité inhibitrice similaire aux composés P2 et P3 (3,1 ± 0,6 µM).

Des analogues halogénés ont également été préparés. Il s'agit des composés P13, P14 et P15. Les composés P13 et P14, dans lesquels un chlore et un fluor sont situés en para de chacun des groupes phényles, respectivement, ont une activité équivalente au composé P3. De manière surprenante, le composé P15, qui est similaire au composé P7 auquel un atome de chlore aurait été substitué aux groupes méthyles en méta de chacun des groupements phényles, présente une activité inhibitrice améliorée avec un Ki d'environ 300 nM.

Les composés P8 et P9 sont des analogues comprenant, en para des groupements phényles des substituants plus volumineux que le groupe méthyle du composés P3, avec respectivement un groupe tert-butyle et n-butyle. Cette modification entraîne une augmentation significative de l'activité inhibitrice des analogues, avec un Ki de 350 ± 40 nM et 641 ± 103 nM respectivement. Il est donc démontré que l'ajout de groupements plus volumineux entraîne une augmentation de l'activité inhibitrice des composés.

Par ailleurs, des analogues comprenant un groupement naphtyle (composé P6), ou benzothiényle (composé P12) à la place de chaque groupement phényle du 2-APB ont été synthétisés. Les composés P6 et P12 présentent une activité inhibitrice importante, avec un Ki de 275 ± 17 nM et 374 ± 96 nM, respectivement.

Cependant, le meilleur inhibiteur identifié est le composé P11, qui comprend un groupement phényle lié en para de chacun des groupements phényles du 2-APB, et présente un Ki de 75 ± 21 nM sur cellules Jurkat. De manière tout à fait surprenante, l'imposition d'une contrainte entre les deux groupements phényles par l'intermédiaire de l'introduction d'un gem-diméthyle (composé P17) entraîne une absence totale d'activité du composé résultant en un Ki supérieur à 3 µM. La rotation libre des deux groupements phényles du composé P11 semble donc un paramètre important pour le maintien de la très grande efficacité dudit composé.

### 2.2. Inhibition spécifique de la SOCE sans impact sur les mécanismes d'efflux du calcium

Pour déterminer si le composé P11 a une action sur SOCE et non pas sur les mécanismes d'efflux du calcium, une expérience de quenching par le Mn²⁺ a été réalisée au moyen de la molécule indo-1. Les ions Mn²⁺ entrent dans les cellules via les SOCC, mais ne peuvent pas être pompés dans le milieu extracellulaire par les ATPases calcium dépendantes de la membrane plasmique (plasma membrane Ca²⁺ ATPases, ou PMCA), l'échangeur de sodium/calcium (Na⁺/Ca²⁺ exchanger, ou NCX) ou renvoyés vers la lumière du RE par les sarcoplasmic-endoplasmic réticulum Ca²⁺ ATPases, ou SERCA). Une fois dans la cellule, Mn²⁺ se lie à indo-1 et quenche sa fluorescence mesurée à 430 nm. Une augmentation de l'amplitude de l'influx de Mn²⁺ est donc associée à une augmentation de la vitesse de quenching de indo-1, et inversement.

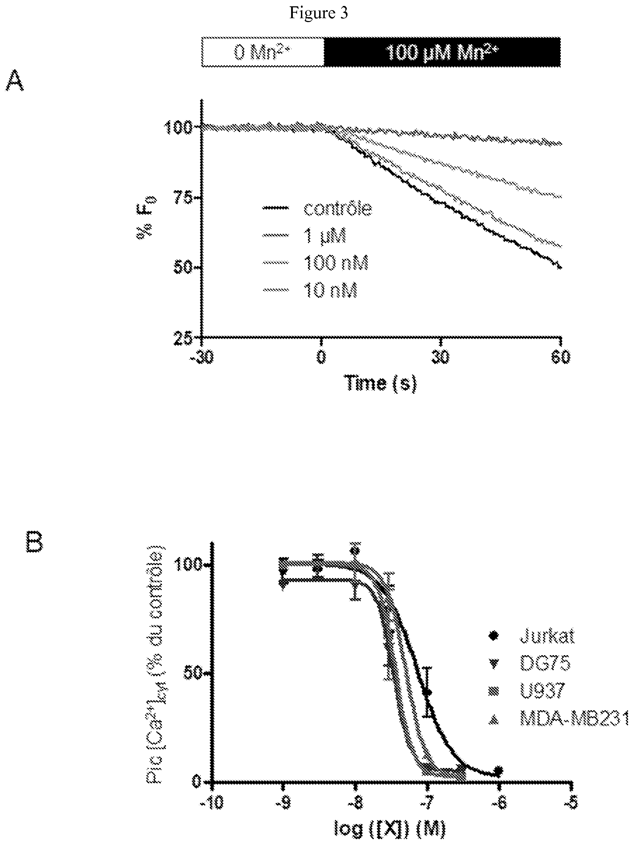

Après 10 minutes de traitement avec TG pour ouvrir les SOCC, 100 µM de MnCl₂ ont été ajoutés. Un taux de quenching de -0,89 ± 0,05 % Fo/s a été observée (figure 3A). En présence de concentrations croissantes de P11 ajouté 30 secondes avant l'ajout d'ions Mn²⁺, le taux de quenching a diminué pour atteindre un blocage d'environ 90% à 1 µM (-0,09 ± 0,01, figure 3A).

Le composé P11 cible donc bien le SOCE.

Par ailleurs, la figure 3B montre que P11 est également capable de bloquer la SOCE dans d'autres types cellulaires, avec une efficacité significativement supérieure à celle observée dans les cellules Jurkat. Ainsi, des Ki de 32 ± 2 nM, 40 ± 5 nM and 50 ± 5 nM ont été calculés dans les cellules DG75 (lymphocytes B), U937 (monocytes) et MDA-MB231 (cancer du sein hormono-indépendant) respectivement, contre 75 ± 21 nM pour les Jurkat cells (figure 3B). La figure 3B montre également qu'une inhibition totale est atteinte à 100 nM.

### 2.3. Sélectivité des composés de l'invention

La sélectivité du composé P11 et du composé proche P9 a été évaluée.

Les mesures de la charge en ⁴⁵Ca²⁺ et la libération dépendant de l'IP3 ont été réalisées sur des cellules perméabilisés dans des conditions unidirectionnelles.

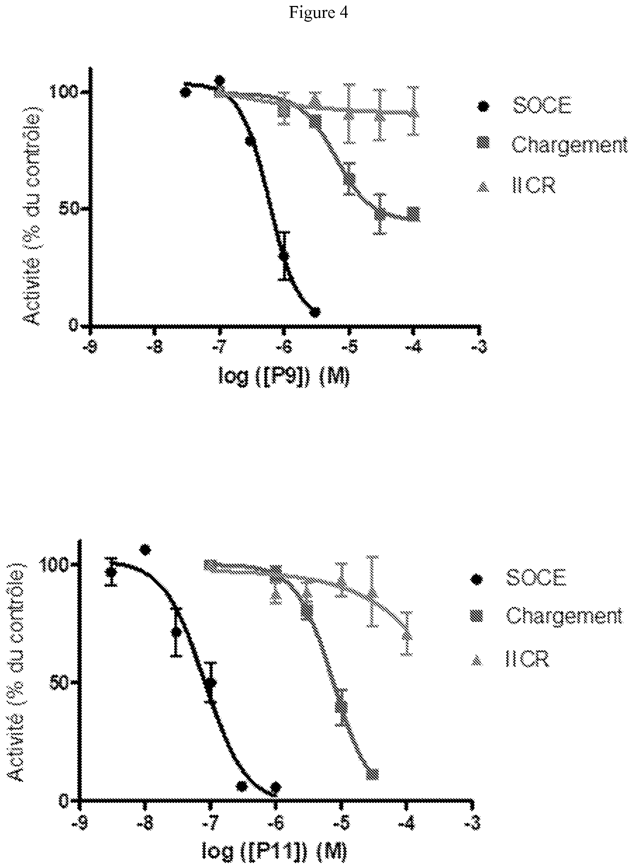

La figure 4 présente les résultats obtenus avec P9 et P11.

P9 n'a pas inhibé de manière significative l'activité de l'IP3R est n'est qu'un inhibiteur partiel de l'activité de SERCA, avec une inhibition de -52 ± 3 % à 100 µM et un Ki de 6 ± 0,3 µM. En dessous de 3 µM, le composé P9 n'inhibe que le SOCE.

P11 n'inhibe que très légèrement l'IP3R, avec une valeur de 29 ± 9% à 100 µM, le calcul du Ki par rapport à ce récepteur s'étant par ailleurs révélé impossible en raison des très fortes concentrations qui auraient été nécessaire pour obtenir cette valeur. SERCA est inhibé à 89% par P11, à une concentration supérieure à 30 µM. Cependant, cette activité est sans commune mesure avec celle observée sur la SOCE, puisque le Ki pour SERCA est de 7,4 µM alors que celui pour SOCE est de 75 nM, soit 100 fois moins pour ce dernier. Il en résulte que P11 est un inhibiteur plus sélectif du SOCE à des concentrations inférieures à 1 µM, sans effet sur SERCA et IP3R.

### 2.4. Les composés de l'invention empêchent l'activation des cellules Jurkat

L'activation des lymphocytes T requiert l'augmentation de la concentration en calcium cytosolique pendant plusieurs minutes pour permettre la synthèse d'interleukine 2 (IL-2), et l'activation des fonctions immunitaires. En conséquence, l'inhibition de l'augmentation de la concentration en calcium cytosolique due à la SOCE empêche l'activation des lymphocytes T et leur prolifération.

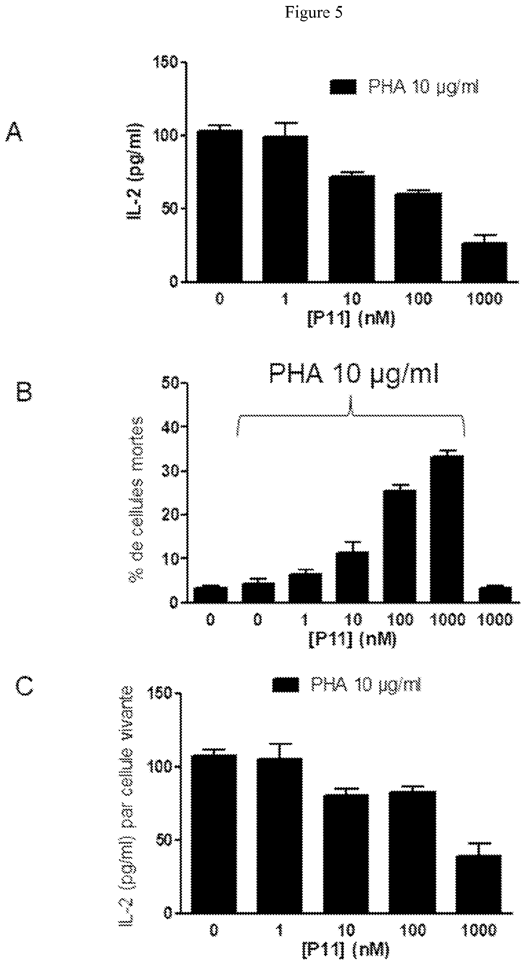

La concentration d'IL-2 néosynthétisée a été mesurée dans le milieu de culture de cellules Jurkat stimulée par de la phytohémaglutinine (PHA) après 24 h, en présence de concentrations croissantes de composé P11. En absence de stimulation par PHA, les cellules Jurkat ne produisent pas d'IL-2. A contrario, une stimulation avec de la PHA permet la détection d'environ 100 pg/ml d'IL-2 (103 ± 4 pg/ml, figure 5A). L'ajout de concentrations croissantes de P11 en même temps que la stimulation par PHA entraîne une diminution progressive de la synthèse d'IL-2 à 26 ± 6 pg/ml pour 1 µM de P11 (- 74%, figure 5B).

La PHA seule ou P11 seul n'ont pas présenté d'effet toxique sur les cellules. Cependant, la combinaison d'un traitement des cellules avec PHA et des concentrations croissantes de P11 augmente significativement le pourcentage de cellules mortes (passant de 5% environ sans traitement, à 33,2 ± 1,3 % à 1 µM de P11, figure 5B). Cependant, le calcul de la quantité d'IL-2 synthétisée par les cellules vivantes restantes montre que des quantités croissantes de P11 diminuent la capacité de synthèse de cette cytokine par les lymphocytes T d'environ 63 %, passant de 107,6 ± 4,1 pg/ml à 39,2 ± 8,6 pg/ml (figure 5C).

P11 a donc un effet double sur les cellules Jurkat activées: une réduction de la synthèse d'IL-2 et une induction de leur mort.

### 2.5. Induction de l'apoptose des cellules Jurkat activées

L'activité de la caspase-3 a ensuite été évaluée au moyen d'un substrat fluorogénique Ac-DEVD-AFC, et l'apoptose a été évaluée par un essai TUNEL.

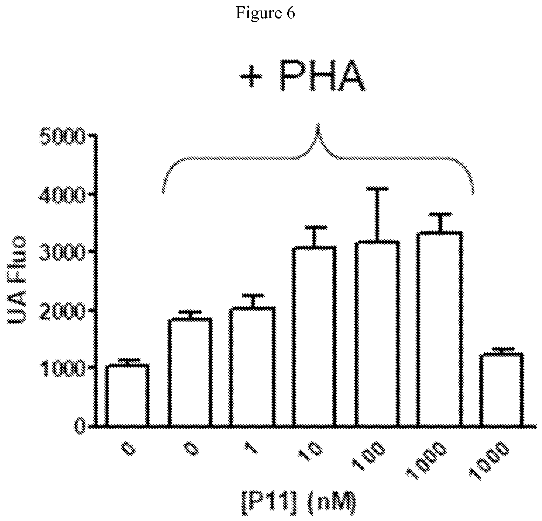

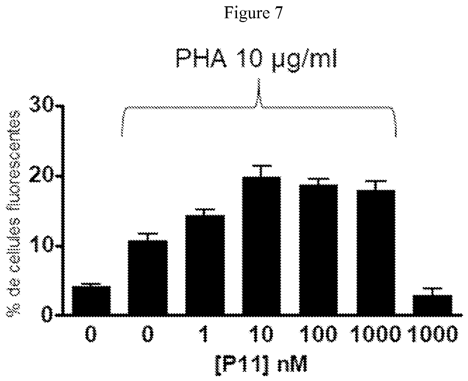

La figure 6 montre que le composé P11 seul n'induit pas une augmentation de l'activité caspase-3, y compris à une concentration de 1 µM. L'activité caspase-3 augmente d'un facteur 2 après stimulation avec de la PHA pendant 24 h (1836 ± 132 unité arbitraires (UA) contre 1037 ± 100 UA). L'ajout supplémentaire de P11, et ce dès 10 nM, entraîne un effet d'activation maximal avec une augmentation d'un facteur 3 (3332 ± 113 UA à 1 µM). Des résultats similaires ont été obtenus au moyen de l'essai TUNEL, qui montre qu'à des concentrations supérieures à 10 nM, le composé P11 induit une augmentation de 4,5 fois du nombre de cellules contenant de l'ADN fragmenté (17,9 ± 1,2 %), dans les cellules stimulées avec de la PHA (figure 7).

Ces résultats montrent donc que le composé P11 ne présente pas d'effet toxique sur les cellules non-activées, mais qu'il a un impact sur l'activation des cellules Jurkat par induction de leur apoptose.

## Revendications

1. Composé de formule (I) ou un sel pharmaceutiquement acceptable de celui-ci, pour son utilisation en tant que médicament: 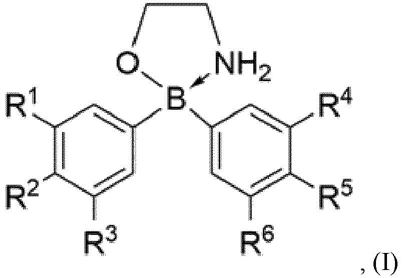
dans laquelle, R1, R2, R3, R4, R5 et R6 sont des groupes identiques ou différents, choisis parmi un atome d'hydrogène, un atome d'halogène, un groupement alkyle en C1-C6, un groupement alcényle en C1-C6, un groupement alkyloxy en C1-C6, un groupement alkylthio en C1-C6, un groupement aryle en C6-C14 et un groupement hétéroaryle comprenant 5 à 14 chaînons;
sous réserve que lorsque deux des groupements R1, R2 et R3 représentent un hydrogène, alors le groupement parmi R1, R2 et R3 qui n'est pas un hydrogène est choisi parmi un groupement alkyle en C3-C6, un groupement alcényle en C3-C6, un groupement alkyloxy en C3-C6, un groupement alkylthio en C3-C6, un groupement aryle en C6-C14 et un groupement hétéroaryle comprenant 5 à 14 chaînons;
sous réserve que lorsque deux des groupements R4, R5 et R6 représentent un hydrogène, alors le groupement parmi R4, R5 et R6 qui n'est pas un hydrogène est choisi parmi un groupement alkyle en C3-C6, un groupement alcényle en C3-C6, un groupement alkyloxy en C3-C6, un groupement alkylthio en C3-C6, un groupement aryle en C6-C14 et un groupement hétéroaryle comprenant 5 à 14 chaînons;
les groupements R1 et R2 pouvant former, avec les atomes de carbone auxquels ils sont liés, un cycle comprenant 5 à 7 chaînons;
les groupements R4 et R5 pouvant former, avec les atomes de carbone auxquels ils sont liés, un cycle comprenant 5 à 7 chaînons; et
lesdits groupements aryles ou hétéroaryles étant susceptibles d'être substitués par un ou plusieurs substituants choisis parmi les atomes d'halogène, les groupements alkyles en C1-C6, les groupements alcényles en C2-C6, les groupements alkyloxy en C1-C6 et les groupements alkylthio en C1-C6, les groupements cétones en C2-C6, les groupements esters en C1-C6, les groupements aldéhydes en C1-C6, -NH2, -CN, -CF₃, les groupements amine en C1-C6 et les groupements amine en C1-C6 mono- ou di-substitués par un groupement alkyle en C1-C6.

2. Composé pour son utilisation selon la revendication 1, dans lequel R1, R3, R4 et R6 représentent un atome d'hydrogène et R2 et R5, identiques, représentent un groupement alkyle en C3-C6, notamment un groupement n-butyle, isobutyle, sec-butyle ou tert-butyle, plus particulièrement n-butyle, ou un groupement aryle en C6-C14, plus particulièrement un groupement aryle en C6-C8.

3. Composé pour son utilisation selon la revendication 2, dans lequel R2 et R5 représentent un groupe phényle substitué ou non-substitué.

4. Composé pour son utilisation selon l'une quelconque des revendications 1 à 3, ledit composé étant la 2-(dibiphényl-4-ylboryloxy)éthamine (composé P11).

5. Composé pour son utilisation selon la revendication 1 ou 2, ledit composé étant la 2-(bis(4-(tert-butyl)phényl)boryl)oxy)éthanamine (composé P9).

6. Composé pour son utilisation selon la revendication 1, dans lequel:
- R3 est un atome d'hydrogène et R1 et R2, identiques ou différents, représentent un atome d'halogène ou un groupement alkyle en C1-C6; et/ou
- R6 est un atome d'hydrogène et R4 et R5, identiques ou différents, représentent un atome d'halogène ou un groupement alkyle en C1-C6.

7. Composé pour son utilisation selon la revendication 1 ou 6, dans lequel:
- R2 représente un atome d'hydrogène, et R1 et R3, identiques ou différents, représentent un atome d'halogène ou un groupement alkyle en C1-C6; et/ou
- R5 est un atome d'hydrogène et R4 et R6, identiques ou différents, représentent un atome d'halogène ou un groupement alkyle en C1-C6.

8. Composé pour son utilisation selon l'une quelconque des revendications 1 à 7, en tant qu'immunosuppresseur.

9. Composé pour son utilisation selon l'une quelconque des revendications 1 à 7, pour le traitement d'un trouble inflammatoire, d'un trouble immunitaire, d'une allergie ou d'un cancer, en particulier un cancer du sein, plus particulièrement un cancer du sein hormono-indépendant.

10. Composé pour son utilisation selon la revendication 9, pour le traitement d'un trouble inflammatoire chronique, d'une leucémie, d'un lymphome, d'une hypertension artérielle pulmonaire ou pour prévenir un rejet de greffe.

## Patentansprüche

1. Verbindung der Formel (I) oder ein pharmazeutisch verträgliches Salz davon zur Verwendung als Arzneimittel: 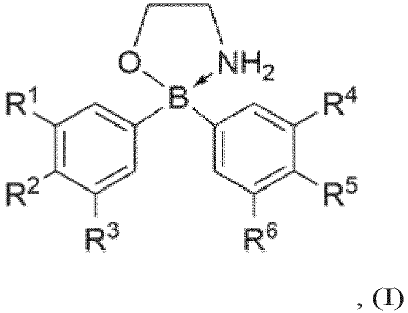
wobei R1, R2, R3, R4, R5 und R6 identische oder unterschiedliche Gruppen sind, ausgewählt aus einem Wasserstoffatom, einem Halogenatom, einer Cl-C6-Alkylgruppe, einer Cl-C6-Alkenylgruppe, einer Cl-C6-Alkyloxygruppe, einer Cl-C6-Alkylthiogruppe, einer C6-Cl4-Arylgruppe und einer Heteroarylgruppe mit 5 bis 14 Ringgliedern;
unter der Bedingung, dass, wenn zwei der Gruppen R1, R2 und R3 Wasserstoff darstellen, die Gruppe von R1, R2 und R3, die kein Wasserstoff ist, aus einer C3-C6-Alkylgruppe, einer C3-C6-Alkenylgruppe, einer C3-C6- Alkyloxygruppe, einer C3-C6-Alkylthiogruppe, einer C6-C14-Arylgruppe und einer Heteroarylgruppe mit 5 bis 14 Ringgliedern ausgewählt wird;
unter der Bedingung, dass, wenn zwei der Gruppen R4, R5 und R6 Wasserstoff darstellen, die Gruppe von R4, R5 und R6, die kein Wasserstoff ist, aus einer C3-C6-Alkylgruppe, einer C3-C6-Alkenylgruppe, einer C3-C6-Alkyloxygruppe, einer C3-C6-Alkylthiogruppe, einer C6-C14-Arylgruppe und einer Heteroarylgruppe mit 5 bis 14 Ringgliedern ausgewählt wird;
wobei die Gruppen R1 und R2 mit den Kohlenstoffatomen, an die sie gebunden sind, einen Ring mit 5 bis 7 Gliedern bilden können;
wobei die Gruppen R4 und R5 mit den Kohlenstoffatomen, an die sie gebunden sind, einen Ring mit 5 bis 7 Gliedern bilden können; und
wobei die Aryl- oder Heteroarylgruppen durch einen oder mehrere Substituenten substituiert werden können, ausgewählt aus Halogenatomen, C1-C6-Alkylgruppen, C2-C6-Alkenylgruppen, Cl-C6-Alkyloxygruppen, und C1-C6 Alkylthiogruppen, C2-C6-Ketongruppen, C1-C6-Estergruppen, Cl-C6-Aldehydgruppen, -NH₂, -CN, -CF₃, Cl-C6-Amingruppen und Cl-C6-Amingruppen, die durch eine Cl-C6-Alkylgruppe mono- oder disubstituiert sind.

2. Verbindung zur Verwendung nach Anspruch 1, in der R1, R3, R4 und R6 ein Wasserstoffatom darstellen und R2 und R5, identisch, eine C3-C6-Alkylgruppe, insbesondere eine n-Butyl-, Isobutyl-, sec-Butyl- oder tert-Butyl-Gruppe, insbesondere n-Butyl, oder eine C6-Cl4-Arylgruppe, insbesondere eine C6-C8-Arylgruppe, darstellen.

3. Verbindung zur Verwendung nach Anspruch 2, in der R2 und R5 eine substituierte oder unsubstituierte Phenylgruppe darstellen.

4. Verbindung zur Verwendung nach einem der Ansprüche 1 bis 3, wobei die Verbindung 2-(Dibiphenyl-4-ylboryloxy)ethamin (Verbindung P11) ist.

5. Verbindung zur Verwendung nach Anspruch 1 oder 2, wobei die Verbindung 2-(Bis(4-(tert-butyl)phenyl)boryl)oxy)ethanamin (Verbindung P9) ist.

6. Verbindung zur Verwendung nach Anspruch 1, wobei:
- R3 ein Wasserstoffatom ist und R1 und R2, gleich oder verschieden, ein Halogenatom oder eine Cl-C6-Alkylgruppe darstellen; und/oder
- R6 ist ein Wasserstoffatom ist und R4 und R5, gleich oder verschieden, ein Halogenatom oder eine Cl-C6-Alkylgruppe darstellen.

7. Verbindung zur Verwendung nach Anspruch 1 oder 6, wobei:
- R2 ein Wasserstoffatom darstellt und R1 und R3, gleich oder verschieden, ein Halogenatom oder eine Cl-C6-Alkylgruppe darstellen; und/oder
- R5 ein Wasserstoffatom ist und R4 und R6, gleich oder verschieden, ein Halogenatom oder eine Cl-C6-Alkylgruppe darstellen.

8. Verbindung zur Verwendung nach einem der Ansprüche 1 bis 7 als Immunsuppressivum.

9. Verbindung zur Verwendung nach einem der Ansprüche 1 bis 7 zur Behandlung einer entzündlichen Erkrankung, einer Immunerkrankung, einer Allergie oder einer Krebserkrankung, insbesondere eines Brustkrebses, insbesondere eines hormonunabhängigen Brustkrebses.

10. Verbindung zur Verwendung nach Anspruch 9 zur Behandlung einer chronisch entzündlichen Erkrankung, Leukämie, Lymphom, pulmonaler arterieller Hypertonie oder zur Verhinderung einer Transplantatabstoßung.

## Claims

1. A compound of formula (I) or a pharmaceutically acceptable salt thereof, for use as a medicament: 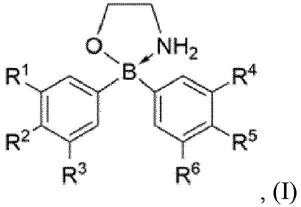
wherein, R1, R2, R3, R4, R5 and R6 are identical or different groups, selected from a hydrogen atom, a halogen atom, a C1-C6 alkyl group, a C1-C6 alkenyl group, a C1-C6 alkyloxy group, a C1-C6 alkylthio group, a C6-C14 aryl group and a heteroaryl group comprising 5 to 14 members;
provided that when two of the groups R1, R2 and R3 represent hydrogen, then the group among R1, R2 and R3 which is not a hydrogen is selected from a C3-C6 alkyl group, a C3-C6 alkenyl group, a C3-C6 alkyloxy group, a C3-C6 alkylthio group, a C6-C14 aryl group and a heteroaryl group comprising 5 to 14 members;
provided that when two of the groups R4, R5 and R6 represent hydrogen, then the group among R4, R5 and R6 which is not hydrogen is selected from a C3-C6 alkyl group, a C3-C6 alkenyl group, a C3-C6 alkyloxy group, a C3-C6 alkylthio group, a C6-C14 aryl group and a heteroaryl group comprising 5 to 14 members;
the groups R1 and R2 being able to form, with the carbon atoms to which they are bound, a ring comprising 5 to 7 members;
the groups R4 and R5 being able to form, with the carbon atoms to which they are bound, a ring comprising 5 to 7 members; and
said aryl or heteroaryl groups being optionally substituted by one or more substituents selected from halogen atoms, C1-C6 alkyl groups, C2-C6 alkenyl groups, C1-C6 alkyloxy groups and C1-C6 alkylthio groups, C2-C6 ketone groups, C1-C6 ester groups, C1-C6 aldehyde groups, -NH2, -CN, -CF3, C1-C6 amine groups and C1-C6 amine groups which are mono- or di-substituted by a C1-C6 alkyl group.

2. The compound for use according to claim 1, wherein R1, R3, R4 and R6 represent a hydrogen atom and R2 and R5, which are identical, represent a C3-C6 alkyl group, in particular an n-butyl, isobutyl, sec-butyl or tert-butyl, more particularly n-butyl, or a C6-C14 aryl group, more particularly a C6-C8 aryl group.

3. The compound for use according to claim 2, wherein R2 and R5 represent a substituted or unsubstituted phenyl group.

4. The compound for use according to any one of claims 1 to 3, said compound being 2-(dibiphenyl-4-ylboryloxy)ethamine (compound P11).

5. The compound for use according to claim 1 or 2, said compound being 2-(bis(4-(tert-butyl)phenyl)boryl)oxy)ethanamine (compound P9).

6. The compound for use according to claim 1, wherein:
- R3 is a hydrogen atom and R1 and R2, which are identical or different, represent a halogen atom or a C1-C6 alkyl group; and/or
- R6 is a hydrogen atom and R4 and R5, which are identical or different, represent a halogen atom or a C1-C6 alkyl group.

7. The compound for use according to claim 1 or 6, wherein:
- R2 represents a hydrogen atom, and R1 and R3, which are identical or different, represent a halogen atom or a C1-C6 alkyl group; and/or
- R5 is a hydrogen atom and R4 and R6, which are identical or different, represent a halogen atom or a C1-C6 alkyl group.

8. The compound for use according to any one of claims 1 to 7, as an immunosuppressant.

9. The compound for use according to any one of claims 1 to 7, for the treatment of an inflammatory disorder, an immune disorder, an allergy or a cancer, in particular a breast cancer, more particularly hormone-independent breast cancer.

10. The compound for use according to claim 9, for the treatment of a chronic inflammatory disorder, leukemia, lymphoma, pulmonary arterial hypertension or to prevent graft rejection.
